(19) Europäisches Patentamt
European Patent Office
Office européen des brevets

(11) EP 2 327 758 A1

(12) **EUROPEAN PATENT APPLICATION**
published in accordance with Art. 153(4) EPC

(43) Date of publication:
**01.06.2011 Bulletin 2011/22**

(21) Application number: **09814535.2**

(22) Date of filing: **11.09.2009**

(51) Int Cl.:
***C11D 11/00** (2006.01)* ***A61K 8/33** (2006.01)*
***A61K 8/37** (2006.01)* ***A61K 8/39** (2006.01)*
***A61Q 5/02** (2006.01)* ***A61Q 19/10** (2006.01)*
***C11D 1/72** (2006.01)* ***C11D 3/20** (2006.01)*

(86) International application number:
**PCT/JP2009/065939**

(87) International publication number:
**WO 2010/032690 (25.03.2010 Gazette 2010/12)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **17.09.2008 JP 2008237954**

(71) Applicant: **Kao Corporation**
**Chuo-ku**
**Tokyo 103-8210 (JP)**

(72) Inventors:
- **HOSOYA,Shingo**
**Wakayama 640-85580 (JP)**
- **ABE,Shuichi**
**Wakayama 640-85580 (JP)**
- **MATSUYAMA,Kazuo**
**Wakayama 640-8580 (JP)**
- **FUKUDA,Kimikazu**
**Wakayama 640-8580 (JP)**
- **MINE,Koji**
**Wakayama 640-85580 (JP)**

(74) Representative: **HOFFMANN EITLE**
**Patent- und Rechtsanwälte**
**Arabellastraße 4**
**81925 München (DE)**

# (54) PEARLESCENT COMPOSITION MANUFACTURING METHOD

(57) A method for producing a pearly luster composition containing a fatty acid glycol ester, a surfactant, and water, and further containing as a crystallization additive any one selected from the group consisting of (1) a fatty acid, (2) an aliphatic alcohol, (3) a fatty acid monoglyceride, and (4) an aliphatic ether, the method including the step of cooling a molten mixture solution containing the fatty acid glycol ester, the surfactant, water, and the crystallization additive, wherein a representative heat-removal rate per unit mass during crystallization in the cooling step is from 9 to 36 [W/kg]. The pearly luster composition obtained by the method of the present invention can be suitably used for shampoos, conditioners, body shampoos, liquid detergents, and the like.

EP 2 327 758 A1

## Description

### TECHNICAL FIELD

The present invention relates to a method for producing a pearly luster composition. More specifically, the present invention relates to a method for producing a pearly luster composition, which can be suitably used to enhance the added values of shampoos, conditioners, body shampoos, liquid detergents, and the like.

### BACKGROUND ART

Conventionally, in order to enhance the added values of shampoos, conditioners, body shampoos, cosmetics, liquid detergents, and the like, a base material giving a pearly luster has been used. As a main component for giving a pearly luster in the pearly luster composition, fatty acid glycol esters, fatty acid monoalkylolamides, fatty acids, and the like have been known (see Patent Publication 1). Among them, various fatty acid glycol esters have been studied as a main component in the pearly luster composition. However, when the amount of a fatty acid glycol ester formulated is increased to obtain a sufficient pearly luster, a viscosity under room temperature is increased, so that free flowability is lowered. Therefore, a pearly luster composition in which a specified nonionic surfactant is used together has been suggested (see Patent Publication 2).

In addition, other base materials giving a pearly luster have been also studied. For example, Patent Publication 3 discloses a pearly luster concentrate containing an aliphatic compound such as an aliphatic alcohol, a fatty acid monoglyceride, or an aliphatic ether, a surfactant, and a polyol, in place of a fatty acid glycol ester. Patent Publication 4 discloses a pearly luster agent concentrate containing an aliphatic alcohol having a very long chain, a fatty acid monoglyceride, an aliphatic ether, or the like.

### PRIOR ART PUBLICATIONS

### PATENT PUBLICATIONS

Patent Publication 1: JP-A-Hei-6-504781
Patent Publication 2: JP2000-212031 A
Patent Publication 3: JP2000-511913 A
Patent Publication 4: JP2003-506393 A

### SUMMARY OF THE INVENTION

The present invention relates to a method for producing a pearly luster composition containing a fatty acid glycol ester, a surfactant, and water, and further containing as a crystallization additive any one selected from the group consisting of (1) a fatty acid, (2) an aliphatic alcohol, (3) a fatty acid monoglyceride, and (4) an aliphatic ether,
the method including the step of cooling a molten mixture solution containing the fatty acid glycol ester, the surfactant, water, and the crystallization additive,
wherein a representative heat-removal rate per unit mass during crystallization in the cooling step is from 9 to 36 [W/kg].

### DETAILED DESCRIPTION OF THE INVENTION

When the pearly luster composition is formulated in cosmetics, detergents, or the like, a pearly luster agent in which a sufficient pearly texture is exhibited with the formulation in an amount as small as possible, in other words, a pearly luster agent having a high whiteness, and excellent dispersion stability has been desired.

Specifically, the present invention relates to a method for producing a pearly luster composition having a high whiteness while maintaining a strong pearly luster, and having even more excellent dispersion stability.

The pearly luster composition obtained by the method of the present invention exhibits some excellent effects that the pearly luster composition has a high whiteness while maintaining strong pearly luster, so that a sufficiently pearly texture is exhibited even with a smaller amount of formulation, and further that the composition has excellent dispersion stability.

The method for producing a pearly luster composition of the present invention is a method for producing a pearly luster composition containing a fatty acid glycol ester, a surfactant, water, and a specified crystallization additive, and the method for producing a pearly luster composition has a great feature in that the method includes the step of

cooling a molten mixture solution containing the fatty acid glycol ester, the surfactant, water, and the specified crystallization additive, wherein a representative heat-removal rate per unit mass during crystallization in the cooling step is controlled within a specified range. Accordingly, although the detailed reasons are not elucidated, fine pearly luster forming particles containing a fatty acid glycol ester, which is a pearly luster forming component, are precipitated in a large amount, so that a pearly luster composition having a high whiteness and excellent dispersion stability is obtained. Here, in the present invention, as a value for showing a whiteness of the pearly luster composition, a W value shown in Examples set forth below can be used. The W value is preferably from 16 to 43, and more preferably from 18 to 40, from the viewpoint of giving luster to the composition.

The fatty acid glycol ester includes, for example, compounds represented by the formula (I):

$$Y\text{-}O\text{-}(CH_2CH_2O)_p\text{-}COR^1 \qquad (I)$$

wherein $R^1$ is a linear or branched, saturated or unsaturated hydrocarbon group having 13 to 21 carbon atoms, Y is a hydrogen atom or $-COR^1$ ($R^1$ is as defined above), and p is the number of from 1 to 3, which means an average number of moles.

In the formula (I), $R^1$ is preferably an alkyl group and an alkenyl group, having 13 to 21 carbon atoms. Specifically, $R^1$ includes a pentadecyl group, a heptadecyl group, a heneicosyl group, and the like. In addition, the fatty acid glycol ester may be either a monocarboxylate ester wherein Y is a hydrogen atom, or a dicarboxylate ester wherein Y is $-COR^1$, as represented by the formula (I). In the dicarboxylate ester, $R^1$ may be identical or different.

As the fatty acid glycol ester, those having a melting point of 50°C or higher are preferable, and those being crystalline are preferable. Therefore, as the fatty acid glycol ester, those having a melting point of 50°C or higher and being crystalline are more preferable. Specifically, the fatty acid glycol ester includes monoethylene glycols such as ethylene glycol monopalmitate, ethylene glycol monostearate, ethylene glycol monoisostearate, ethylene glycol dipalmitate, ethylene glycol distearate, and ethylene glycol dibehenate; diethylene glycols thereof; and triethylene glycols thereof; and the like. Those fatty acid glycol esters may be used alone or in admixture of two or more kinds.

Incidentally, when the fatty acid glycol esters are used in admixture of two or more kinds, the fatty acid glycol ester may be a mixture of the fatty acid glycol esters each prepared, and may be a mixture of the fatty acid glycol esters obtained by a reaction using a mixture of fatty acids having different lengths of the alkyl chains and glycol. For example, from the reaction of a mixture of palmitic acid and stearic acid with glycol, a mixture of ethylene glycol dipalmitate, ethylene glycol monopalmitate and monostearate, and ethylene glycol distearate is obtained. In the mixture of fatty acids used upon the reaction of the mixture of different fatty acids with glycol, the percentage of each fatty acid is preferably 85% by weight or less.

In the fatty acid glycol esters exemplified above, as the preferable fatty acid glycol esters in the present invention, ethylene glycol distearate, ethylene glycol dipalmitate, ethylene glycol monostearate, ethylene glycol monopalmitate, and ethylene glycol dibehenate, and a mixture of ethylene glycol dipalmitate, ethylene glycol monopalmitate and monostearate, and ethylene glycol distearate, are preferable.

The fatty acid glycol ester is contained in the pearly luster composition in an amount of preferably 15% by weight or more from the viewpoint of giving the pearly luster, and preferably 30% by weight or less from the viewpoint of free flowability. From the above viewpoints, the fatty acid glycol ester is contained in an amount of preferably from 15 to 30% by weight, more preferably from 15 to 25% by weight, and even more preferably from 18 to 25% by weight, of the pearly luster composition.

The surfactant is effective in promoting emulsification of the pearly luster composition, and a nonionic surfactant and an anionic surfactant are suitably used.

The nonionic surfactant includes a polyoxyalkylene nonionic surfactant, a fatty acid monoalkylolamide, and the like.

The polyoxyalkylene nonionic surfactant refers to those having a polyoxyalkylene group such as a polyoxyethylene group or a polyoxypropylene group. Specific examples of the polyoxyalkylene nonionic surfactant include polyoxyalkylene alkyl ethers, polyoxyalkylene alkylphenyl ethers, polyoxyalkylene glycol fatty acid esters, polyoxyalkylene fatty acid esters, polyoxyalkylene sorbitan fatty acid esters, polyoxyalkylene fatty acid monoalkanolamides, polyoxyalkylene fatty acid dialkanolamides, and the like. Those polyoxyalkylene nonionic surfactants may be used alone or in admixture of two or more kinds. Among them, polyoxyalkylene alkyl ethers represented by the formula (II):

$$R^2\text{-}O\text{-}(R^3O)_q\text{-}H \qquad (II)$$

wherein $R^2$ is a linear or branched, saturated or unsaturated hydrocarbon group having 8 to 20 carbon atoms, $R^3$ is an ethylene group or a propylene group, and q is the number of from 1 to 12 and preferably from 1 to 6, which means an average number of moles, are preferable.

In the formula (II), $R^2$ is preferably an alkyl group having 8 to 20 carbon atoms or an alkenyl group having 8 to

20 carbon atoms. In addition, $R^3$ includes an ethylene group, an n-propylene group, and an iso-propylene group. q is preferably from 3 to 6.

The polyoxyalkylene nonionic surfactant has an HLB value of preferably less than 15, and more preferably from 9 to 12.5, from the viewpoint of suppressing an emulsification of the pearly luster composition and controlling the viscosity. Here, the HLB value is an index showing a hydrophilic-lipophilic balance. In the present invention, the HLB value is a value calculated using the equation according to Oda and Teramura, et al.:

$$HLB = (\Sigma \text{ Inorganic Value}/\Sigma \text{ Organic Value}) \times 10$$

The polyoxyalkylene nonionic surfactant is contained in the pearly luster composition in an amount of preferably 0.5% by weight or more from the viewpoint of lowering the viscosity of the pearly luster composition, and preferably 10% by weight or less from the viewpoint of obtaining an excellent pearly luster. From the above viewpoints, the polyoxyalkylene nonionic surfactant is contained in an amount of preferably from 0.5 to 10% by weight, more preferably from 0.5 to 8% by weight, and even more preferably from 1 to 5% by weight, of the pearly luster composition.

The fatty acid monoalkylolamide is effective in an increase in the luster, and includes, for example, a compound represented by the formula (III):

$$R^4CO\text{-}NH\text{-}R^5OH \qquad (III)$$

wherein $R^4$ is a linear or branched, saturated or unsaturated hydrocarbon group having 7 to 20 carbon atoms, and $R^5$ is an ethylene group or a propylene group.

In the formula (III), $R^4$ is preferably an alkyl group and an alkenyl group, having 7 to 20 carbon atoms. Specifically, $R^4$ includes an undecyl group, a tridecyl group, a heptadecyl group, and the like. Also, $R^5$ includes an ethylene group, an n-propylene group, and an iso-propylene group.

The fatty acid monoalkylolamide includes lauric acid monoethanolamide, lauric acid monopropanolamide, lauric acid monoisopropanolamide, myristic acid monoethanolamide, palmitic acid monoethanolamide, stearic acid monoethanolamide, oleic acid monoethanolamide, oleic acid monoisopropanolamide, coconut oil fatty acid monoethanolamide, coconut oil fatty acid monopropanolamide, coconut oil fatty acid monoisopropanolamide, palm vegetable oil fatty acid monoethanolamide, and the like. Those fatty acid monoalkylolamides can be used alone or in admixture of two or more kinds. Among them, coconut oil fatty acid monoethanolamide, lauric acid monoethanolamide, palmitic acid monoethanolamide, and stearic acid monoethanolamide, are preferable.

The fatty acid monoalkylolamide is contained in the pearly luster composition in an amount of preferably 3% by weight or more from the viewpoint of giving a luster, and preferably 15% by weight or less from the viewpoint of suppressing an increase in the viscosity of the pearly luster composition and increasing free flowability. From the above viewpoints, the fatty acid monoalkylolamide is contained in an amount of preferably from 3 to 15% by weight, more preferably from 3 to 10% by weight, and even more preferably from 5 to 10% by weight, of the pearly luster composition.

The anionic surfactant includes fatty acid salts, alkyl sulfates, polyoxyalkylene alkyl ether sulfates, sulfosuccinate surfactants, polyoxyalkylene alkylamido ether sulfates, monoglyceride sulfates, olefin sulfonates, alkylbenzenesulfonates, alkanesulfonates, acyl isethionates, acyl amino acids, alkyl phosphates, polyoxyalkylene alkyl ether phosphates, polyoxyalkylene alkyl ether carboxylates, and the like. Among them, alkyl sulfates are preferable.

Examples of the alkyl sulfates include alkyl sulfates which may have a polyoxyalkylene group represented by the formula (IV):

$$R^6\text{-}O\text{-}(R^7O)_r\text{-}SO_3M \qquad (IV)$$

wherein $R^6$ is a linear or branched, saturated or unsaturated hydrocarbon group having 8 to 20 carbon atoms, $R^7$ is an ethylene group or a propylene group, M is an alkali metal, an alkaline-earth metal, an ammonium ion, or a hydroxyalkyl-substituted ammonium having 2 or 3 carbon atoms, and r is the number of from 0 to 8, which means an average number of moles, and the like.

In the formula (IV), $R^6$ is preferably an alkyl group or an alkenyl group each having 8 to 20 carbon atoms, and specifically includes a lauryl group, a myristyl group, a palmityl group, a stearyl group, and the like. $R^7$ includes an ethylene group, an n-propylene group, and an iso-propylene group. r is preferably from 0 to 4.

Preferred examples of the alkyl sulfates include sodium lauryl sulfate, triethanolamine lauryl sulfate, sodium polyoxyethylene lauryl ether sulfate (the average number of moles of ethylene oxide (EO): 1 to 4), triethanolamine polyoxyethylene lauryl ether sulfate (the average number of moles of EO: 1 to 4), and the like. These alkyl sulfates can be used alone or in admixture of two or more kinds.

The alkyl sulfate is contained in the pearly luster composition in an amount of preferably 5% by weight or more from the viewpoint of homogenously mixing each component, and preferably 15% by weight or less from the viewpoint of free flowability. From these viewpoints, the alkyl sulfate is contained in an amount of preferably from 5 to 15% by weight, more preferably from 8 to 15% by weight, and even more preferably from 8 to 13% by weight.
The fatty acid glycol ester and the surfactant are contained in a total amount of preferably from 25 to 70% by weight, and more preferably from 30 to 60% by weight, of the pearly luster composition.
The water is contained in an amount of preferably from 25 to 75% by weight, more preferably from 40 to 75% by weight, and even more preferably from 50 to 75% by weight, of the pearly luster composition, from the viewpoint of adjustments of the concentration and the viscosity of the pearly luster composition.
In the present invention, as the crystallization additive, any one selected from the group consisting of (1) a fatty acid, (2) an aliphatic alcohol, (3) a fatty acid monoglyceride, and (4) an aliphatic ether is used.
The fatty acid is preferably a saturated or unsaturated fatty acid having 8 to 22 carbon atoms, and may be either linear or branched. Fatty acids having 12 to 18 carbon atoms such as lauric acid, myristic acid, palmitic acid, and stearic acid are more preferable, from the viewpoint of making the crystals finer. Those fatty acids may be used alone or in admixture of two or more kinds.
The aliphatic alcohol is preferably a saturated or unsaturated aliphatic alcohol having 8 to 22 carbon atoms, and may be either linear or branched. Aliphatic alcohols having 12 to 22 carbon atoms such as lauryl alcohol, myristyl alcohol, cetyl alcohol, stearyl alcohol, and behenyl alcohol are more preferable, and aliphatic alcohols having 12 to 18 carbon atoms are even more preferable, from the viewpoint of making the crystals finer. These aliphatic alcohols may be used alone or in admixture of two or more kinds.
The fatty acid monoglyceride is preferably a compound which is an ester of glycerol and a fatty acid, represented by the formula (A):
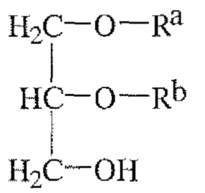

(A)

wherein either one of $R^a$ and $R^b$ is a hydrogen atom and the other is $-COR^c$, wherein $R^c$ is an alkyl group or alkenyl group having 7 to 21 carbon atoms.
In $R^c$, the number of carbon atoms of the alkyl group and the alkenyl group is preferably from 11 to 17. The alkyl group and the alkenyl group may be either linear or branched.
Preferred examples of the fatty acid monoglyceride in the present invention include lauric acid monoglyceride, myristic acid monoglyceride, palmitic acid monoglyceride, stearic acid monoglyceride, behenic acid monoglyceride, coconut oil fatty acid monoglyceride, palm kernel oil fatty acid monoglyceride, tallow fatty acid monoglyceride, mixtures thereof, and the like. The fatty acid monoglyceride may contain small amounts of diglyceride and triglyceride from the manufacturing process.
The aliphatic ether is preferably a compound represented by the formula (B):

$$R^d\text{-O-}R^e \qquad \text{(B)}$$

wherein $R^d$ and $R^e$ are each independently an alkyl group or alkenyl group having 8 to 22 carbon atoms.
In $R^d$ and $R^e$, the number of carbon atoms of the alkyl group and the alkenyl group is preferably from 12 to 18. Also, the alkyl group and the alkenyl group may be either linear or branched. In addition, the aliphatic ether may be either a single ether or a mixed ether, and thus $R^a$ and $R^e$ may be identical to or different from each other.
Preferred examples of the aliphatic ether in the present invention include dilauryl ether, dimyristyl ether, dicetyl ether, distearyl ether, and the like.
The crystallization additive is contained in an amount of preferably from 0.3 to 3% by weight, and more preferably from 0.5 to 2.1% by weight, of the pearly luster composition, from the viewpoint of preventing the deterioration of luster and the lowering of the turbidity caused by excessive formation of fine crystals. In addition, the above crystallization additive is contained in an amount of preferably from 1 to 20 parts by weight, more preferably from 1.5 to 20 parts by weight, even more preferably from 1.5 to 15 parts by weight, and even more preferably from 3 to 10 parts by weight,

based on 100 parts by weight of the fatty acid glycol ester set forth below.

The fatty acid glycol ester, the surfactant, water, the crystallization additive, other additives, and the like can be used in the same manner as mentioned above. In a case where a fatty acid and an aliphatic alcohol are used as crystallization additives, it is preferable that a polyoxyalkylene nonionic surfactant is formulated together therewith. The viscosity can be lowered by the polyoxyalkylene nonionic surfactant, so that not only a strong pearly luster is obtained without impairing free flowability, but also an improvement in turbidity is exhibited.

Further, in the pearly luster composition of the present invention, a pH adjusting agent, a preservative, salts, alcohols, polyols or the like, may be properly formulated.

In the method for producing a pearly luster composition of the present invention, the molten mixture solution is not particularly limited as long as the molten mixture solution is obtained by a method of melting raw materials such as a fatty acid glycol ester and a crystallization additive. The specific method includes, for example, a method including the step of heating the mixture of raw materials such as a fatty acid glycol ester, a crystallization additive, a surfactant, and water; a method including the step of mixing a mixture containing water, a surfactant, and the like, with a fatty acid glycol ester and a crystallization additive in a molten state, and the like.

In addition, the fatty acid glycol ester and the crystallization additive may be added thereto as a mixture solution of both compounds each heated to melt or may be separately added thereto. It is preferable that the molten mixture solution of both compounds is added thereto.

The temperature of the molten mixture solution of the raw materials is preferably a temperature not less than the melting point of the fatty acid glycol ester or the crystallization additive, the melting point of which is the higher of the two, and preferably a temperature not more than the boiling point of the mixture. In addition, the temperature of the molten mixture solution of the raw materials is a temperature higher than the melting point of the fatty acid glycol ester or the crystallization additive, more preferably by from 1° to 30°C, and even more preferably by from 1° to 20°C, the melting point of which is the higher of the two.

The temperature at which the cooling step is terminated is preferably a temperature lower than the melting point of the fatty acid glycol ester, more preferably a temperature equal to or lower than a temperature calculated from the melting point minus 10°C, and even more preferably a temperature equal to or lower than a temperature calculated from the melting point minus 20°C, from the viewpoint of sufficiently crystallizing the fatty acid glycol ester. Furthermore, the temperature at which the cooling step is terminated is preferably a temperature equal to or lower than a temperature calculated from the melting point of the fatty acid glycol ester or the crystallization additive minus 10°C, and more preferably a temperature equal to or lower than a temperature calculated from the melting point minus 20°C, the melting point of which is lower of the two.

In addition, the cooling is preferably a slow cooling with a narrow temperature distribution from the viewpoint of obtaining pearly luster-forming particles having even shapes. From the above viewpoint, the cooling rate is preferably from 0.1° to 10°C/min, more preferably from 0.1° to 5°C/min, and even more preferably from 0.1° to 3°C/min.

It is preferable that cooling is carried out by a method of smaller temperature distribution. A specific method includes, for example, a method including the steps of preparing a molten mixture solution in a formulation tank equipped with a jacket, and allowing cooling water to flow through the jacket, and the like.

During the cooling, the fatty acid glycol ester is crystallized to generate heat of crystallization. The present invention has a feature that a heat-removal rate upon generation of heat of crystallization is controlled.

It is considered that the generation of heat of crystallization eases an overcooling degree, thereby moderating the progress of crystallization. However, it is considered that if a heat-removal procedure is carried out upon heat generation, the ease in the overcooking degree is moderated, and the crystallization is progressed, whereby fine crystals can be obtained. The pearly luster composition having fine crystals has a high whiteness while maintaining a strong pearly luster, and further has high dispersion stability. However, an excessive heat-removal leads to excessively form fine crystals, thereby deteriorating luster. In view of the above, in the present invention, as an index which can easily adjust the progress of crystallization, a representative heat-removal rate per unit mass during the crystallization is found.

The heat-removal rate during the generation of heat of crystallization changes with time due to a temperature change in the pearly luster composition or the like. In view of the above, a midpoint in time from the beginning of crystallization to the termination of crystallization is handled as a representative point, and a temperature of a pearly luster composition at the representative point is handled as a representative midpoint temperature, and the heat-removal rate at the representative point is obtained.

The time points of the beginning and the termination of crystallization can be confirmed by a temperature of a pearly luster composition, a stirring electric current value, an electroconductivity, visual observation, or the like.

The heat-removal rate Q[W] at a representative point is obtained by the formula (X):

$$Q = UA\Delta T \qquad (X)$$

where Q: a heat-removal rate [W],
U: an overall heat-transfer coefficient [W/m$^2$/K],
A: a heat-transfer area [m$^2$], and
$\Delta T$: an average temperature difference [K].
Here, the formula (X) is a general formula expressing heat transfer, as shown in, for example, Kagaku Kogaku Gairon (Introduction to Chemical Engineering) (edited by Atsuro MIZUSHINA and Ryozo TOEI, Sangyo Tosho K.K., Heisei-5 (1993) (15th Printing), page 66).

In the formula (X), $\Delta T$ is obtained from the formula(Y):

$$\Delta T = \frac{(Tp - Tc_1) - (Tp - Tc_2)}{LN[(Tp - Tc_1)/(Tp - Tc_2)]} \quad (Y)$$

where Tp: a temperature [°C] of a pearly luster composition at a representative point (a representative midpoint temperature),
$Tc_1$: a coolant inlet temperature [°C] at the representative point, and
$Tc_2$: a coolant outlet temperature [°C] at the representative point.

Further, U is obtained by the following formula (Z-1) and formula (Z-2).

Supposing that a certain time point in which a cooling step is carried out is defined as a beginning point (0 s), $t_1/2$ [s], which is one-half the amount of $t_1$, when the crystallization begins after $t_1$[S], is defined as $t_2$ [s]. A slope **a** [C/s] of a regression line obtained by measuring temperatures of a pearly luster composition from 0 to $t_1$ seconds in given time intervals indicates a cooling rate and takes a negative value. When the slope **a**[°C/s] of the regression line is obtained, the larger the number of the measured temperature data, the higher the accuracy. For this reason, the temperatures of the pearly luster composition are measured every one second. An overall heat-transfer coefficient $U_{12}$ at $t_2$ [s] is derived from the formula (Z-1) using **a** [°C/s].

$$U_{t2} = -\mathbf{a}Mc/(A\Delta T_{t2}) \quad (Z\text{-}1)$$

where M: an amount [kg] of a pearly luster composition formulated,
c: a specific heat [J/kg/K] of the pearly luster composition,
A: a heat-transfer area [m$^2$], and
$\Delta T_{12}$: an average temperature difference [K] at a time point $t_2$[S].
Here, by making $t_2$ [s], in other words, $t_1$ [s] a short time period, the resulting $U_{12}$ can approximate U in the formula (X). However, if $t_1$[S] is an exceedingly short time period, errors are likely to be caused, and if it is a long time period, the difference of $U_{t2}$ with U in the formula (X) becomes large. Therefore, $U_{t2}$ where $t_1$[S] is 300 seconds is defined as an overall heat-transfer coefficient U in the formula (X).

$$\Delta T_{t2} = \frac{(Tp_{t2} - Tc_{t2\cdot1}) - (Tp_{t2} - Tc_{t2\cdot2})}{LN[(Tp_{t2} - Tc_{t2\cdot1})/(Tp_{t2} - Tc_{t2\cdot2})]} \quad (Z\text{-}2)$$

where $Tp_{t2}$: a temperature [°C] of a pearly luster composition at a time point $t_2$ [S],
$Tc_{12\cdot1}$: a coolant inlet temperature [°C] at a time point $t_2$ [s], and
$Tc_{12\cdot2}$: a coolant outlet temperature [°C] at a time point $t_2$[S].

A value obtained by dividing the heat-removal rate Q[W] at a representative point by an amount of a pearly luster composition formulated M [kg], i.e. Q/M[W/kg], is defined as a representative heat-removal rate per unit mass during the crystallization in the cooling step.

In the production of the pearly luster composition formulated with a crystallization additive, a representative heat-removal rate per unit mass during crystallization is from 9 to 36[W/kg], preferably from 11 to 36 [W/kg], and more preferably from 13 to 36 [W/kg]. A method of controlling the representative heat-removal rate to a specified range can be carried out by, for example, properly adjusting a temperature of cooling water passing though a jacket attached to a formulation tank. In that case, a heat-removal rate upon generation of heat of crystallization of the fatty acid glycol ester

may be controlled, so that the temperature of the cooling water may be adjusted regardless of the cooling rate in the cooling step. The temperature of the cooling water may vary around the representative point, or the temperature may be substantially the same.

After the pearly luster-forming particles are crystallized, it is preferable that the solution is further cooled to stabilize the crystals. It is desired that the solution is cooled until a temperature of the solution is from 10° to 40°C, and preferably from 15° to 35°C.

It is preferable that the raw materials are molten and cooled while stirring so that the solution is not separated.

The size of the formulation tank is not particularly limited, and for example, a formulation tank of from 0.3 L to 20 $m^3$ can be used. In a case of mass-producing in an industrial scale, it is preferable to use a formulation tank having a size of from 100 L to 20 $m^3$. In a case where a formulation tank of an industrial scale as mentioned above is used, it is usually difficult to control the cooling rate to a desired range over an entire cooling step, and consequently a great load would be applied to a cooling facility attached to the tank. In the present invention, the cooling rate is not needed to be always controlled, and a pearly luster composition having a high whiteness and excellent dispersion stability can be conveniently obtained by simply adjusting a representative heat-removal rate per unit mass during the crystallization to a specified range.

## EXAMPLES

The following examples further describe and demonstrate embodiments of the present invention. The examples are given solely for the purposes of illustration and are not to be construed as limitations of the present invention.

Various properties of the pearly luster compositions obtained in each of Examples and each of Comparative Examples were determined according to the following methods.

< Pearly Luster of Pearly Luster Composition >

The pearly luster composition is diluted 20-folds (weight ratio) with water, and an external appearance of the pearly luster is observed with naked eyes, and evaluated according to the following criteria. Incidentally, a composition in which bubbles are commingled is centrifuged to remove bubbles.

< Evaluation Criteria >

A: A strong luster is found.
B: A weak luster is found.
C: No luster is found.

< Whiteness of Pearly Luster Composition >

A dilution prepared by diluting a pearly luster composition 33.3-folds (weight ratio) with an aqueous solution containing 19.5% by weight sodium polyoxyethylene(2) lauryl ether sulfate is weighed in an amount of 1 g and placed in a cell, and L(brightness) and b(hue, chroma) are determined with a colorimeter (SE-2000, manufactured by JEOL Ltd.), to obtain a whiteness according to the following formula as defined by ASTM (American Society for Testing and Materials) (E-313).

$$\text{W value} = (7L^2 - 40Lb)/700$$

wherein a W value is a whiteness of the pearly luster composition, which in other words is used as an index expressing turbidity. The higher the W value, the pearly luster composition becomes white and is concentrated.

< Dispersion Stability of Pearly Luster Composition >

A pearly luster composition was diluted 50-folds (weight ratio) by mixing the composition with an aqueous sodium chloride solution adjusted to have a certain specific gravity (1.028 g/$cm^3$, 1.035 g/$cm^3$, 1.042g/$cm^3$, or 1.049g/$cm^3$) using sodium chloride and water. Twenty-five milliliters of this dilution was added to a test tube having a diameter of 18 mm and a height of 180 mm, and allowed to stand at 23°C for 18 hours. Thereafter, the dispersion stability is evaluated

in accordance with the following criteria. The measurements are taken at 23°C.

[Evaluation Criteria]

A: A composition is stably dispersed while maintaining white turbidity even after having allowed to stand for 18 hours in a 1.028 g/cm$^3$ aqueous sodium chloride solution and a 1.049 g/cm$^3$ aqueous sodium chloride solution.

B: A transparent separation layer is generated after allowing a composition to stand for 18 hours in at least either one of the 1.028 g/cm$^3$ aqueous sodium chloride solution and the 1.049 g/cm$^3$ aqueous sodium chloride solution, so that the composition cannot be stably dispersed; on the other hand, a composition is stably dispersed while maintaining white turbidity even after having allowed to stand for 18 hours in a 1.035 g/cm$^3$ aqueous sodium chloride solution and a 1.042 g/cm$^3$ aqueous sodium chloride solution.

C: A transparent separation layer is generated after allowing a composition to stand for 18 hours in at least either one of the 1.035 g/cm$^3$ aqueous sodium chloride solution and the 1.042 g/cm$^3$ aqueous sodium chloride solution, so that the composition cannot be stably dispersed.

< Melting Point of Fatty Acid Glycol Ester or Crystallization Additive >

The fatty acid glycol ester or the crystallization additive is heated so as to raise the temperature at a rate of 5°C/min using a differential scanning calorimeter (Thermo plus DSC 8230, manufactured by Rigaku Corporation), and the top of the resulting melting peak is defined as a melting point.

< Specific Heat of Pearly Luster Composition >

A DSC curve during heating at uniform velocity is obtained using a differential scanning calorimeter (Thermo plus DSC 8230, manufactured by Rigaku Corporation) for an empty vessel, a sample having a known specific heat, and a molten mixture solution of a pearly luster composition, and a specific heat is obtained by a proportional calculation from an amount shifted from each of the baselines.

<u>Examples 1 to 7 and Comparative Examples 1 and 2</u>

A mixture of a fatty acid monoalkylolamide, an alkyl sulfate, a polyoxyalkylene nonionic surfactant, and other ingredients listed in Tables 1 and 2 was mixed at 80°C with T.K. AGI HOMO MIXER f model (manufactured by PRIMIX Corporation, 2 L specification) in which an agitation rotational speed is set at a rate listed in Tables 1 and 2. Thereto were added a fatty acid glycol ester and a crystallization additive which were previously melted and mixed in a molten state, and the mixture obtained was mixed to provide a molten mixture solution. Thereafter, a cooling water set as shown in Tables 1 and 2 was allowed to flow through a jacket to cool the molten mixture solution, to provide a pearly luster composition.

Here, in Examples and Comparative Examples, an initial crystallization temperature is defined as a temperature at which a liquid temperature begins to increase, and a terminal crystallization temperature is defined as a temperature at which an increase in a liquid temperature is stopped. Further, a midpoint of these temperatures is defined as a representative midpoint temperature.

In addition, an overall heat-transfer coefficient U was calculated according to the formula (Z-1) and the formula (Z-2) mentioned above. In other words, in Example 1 where T.K. AGI HOMO MIXER f model (manufactured by PIZIMIX Corporation, 2 L specification) was used, supposing that a (a slope of a regression curve of a temperature of a pearly luster composition from 0 to $t_1$ seconds for every second, namely cooling rate) = -0.00694°C/s, M (amount of a pearly luster composition formulated) = 2.0 kg, c (specific heat) = 3,090 J/Kg/K, A (heat-transfer area) = 0.072 (m$^2$), $t_1$ = 300 seconds, $t_2$ = 150 seconds, $Tp_{t2}$ = 41.6°C, $Tc_{t2\cdot 1}$ = 35.9°C, $Tc_{t2\cdot 2}$ = 37.5°C were substituted into the formulas, U = 120 W/m$^2$/K was obtained. From the above, U in Examples 1 to 7 and Comparative Examples 1 and 2 in which T.K. AGI HOMO MIXER f model (manufactured by PRIMIX Corporation, 2 L specification) was used was assumed to be 120 W/m$^2$/K.

In Example 8 given later in which T.K. AGI HOMO MIXER S100 model (manufactured by PRIMIX Corporation) was used, supposing that a (a slope of a regression curve of a temperature of a pearly luster composition from 0 to $t_1$ seconds for every second, namely cooling rate) = -0.00528°C/s, M (amount of a pearly luster composition formulated) = 100 kg, c (specific heat) = 3,090 J/Kg/K, A (heat-transfer area) = 1.2 (m$^2$), $t_1$ = 300 seconds, $t_2$ = 150 seconds, $Tp_{t2}$ = 35.2°C, $Tc_{t2\cdot 1}$ = 27.2°C, $Tc_{t2\cdot 2}$ = 29.1°C were substituted into the formulas, U = 190 W/m$^2$/K was obtained. From the above, U in Examples 8 to 15 in which T.K. AGI HOMO MIXER model S100 (manufactured by PRIMIX Corporation, 2

L specification) was used was assumed to be 190 $W/m^2/K$.

[Table 1]

| | | Examples | | | |
|---|---|---|---|---|---|
| | | 1 | 2 | 3 | 4 |
| Mixing Apparatus | | AGIHOMO MIXER (2L) | AGI HOMO MIXER (2L) | AGI HOMO MIXER (2L) | AGI HOMO MIXER (2L) |
| (A) | Fatty Acid Glycol Ester: | | | | |
| | Ethylene Glycol of Difatty Acid (C16/C18 50/50[1)]) | 18.8 | 18.5 | 18.2 | 18.2 |
| (B) | Fatty Acid Monoalkylolamide: | | | | |
| | Coconut Oil Fatty Acid Monoethanolamide | 7.5 | 7.5 | 7.5 | 7.5 |
| (C) | Alkyl Sulfate | | | | |
| | Sodium Polyoxyethylene(2) Lauryl Ether Sulfate[2)] | 11.0 | 11.0 | 11.0 | 11.0 |
| (D) | Polyoxyalkylene Nonionic Surfactant: | | | | |
| | Polyoxyethylene(4) Lauryl Ether[2)] [HLB: 9.7] | 4.0 | 4.0 | 4.0 | 4.0 |
| (E) | Other Ingredients: | | | | |
| | Citric Acid Monohydrate | 0.13 | 0.13 | 0.13 | 0.13 |
| | Sodium Benzoate | 1.0 | 1.0 | 1.0 | 1.0 |
| | Water | Balance | Balance | Balance | Balance |
| (F) | Crystallization Additive | | | | |
| | Fatty Acid[3)] | 1.2 | 1.5 | 1.8 | 1.8 |
| M: | Amount of Pearly Luster Composition Formulated (kg) | 2.0 | 2.0 | 2.0 | 2.0 |
| Agitation Rotational Speed (r/min) | | 46 | 46 | 46 | 120 |
| c: | Specific Heat of Pearly Luster Composition (J/kg/K) | 3,090 | 3,090 | 3,090 | 3,090 |
| U: | Overall Heat-Transfer Coefficient ($W/m^2/K$) | 120 | 120 | 120 | 120 |

(continued)

| Mixing Apparatus | | AGIHOMO MIXER (2L) | AGI HOMO MIXER (2L) | AGI HOMO MIXER (2L) | AGI HOMO MIXER (2L) |
|---|---|---|---|---|---|
| Setting of Cooling Water | | Cooling at 0.5°C/min | Cooling at 0.1°C/min | Cooling at 0.1°C/min Keeping | Cooling at 0.5°C/min and at 32°C |
| Initial Crystallization Temperature (°C) | | 40.4 | 43.5 | 35.8 | 34.0 |
| Terminal Crystallization Temperature (°C) | | 45.1 | 48.2 | 40.4 | 36.7 |
| Tp: | Representative Midpoint Temperature (°C) | 42.8 | 45.9 | 38.1 | 35.4 |
| $Tc_1$: | Coolant Temperature (°C) of Jacket Inlet at Representative Point | 34.0 | 41.1 | 33.3 | 32.1 |
| $Tc_2$: | Coolant Temperature (°C) of Jacket Outlet at Representative Point | 36.1 | 41.5 | 33.9 | 32.0 |
| ΔT: | Average Temperature Difference (K) | 7.7 | 4.6 | 4.5 | 3.3 |
| A: | Heat-Transfer Area ($m^2$) | 0.072 | 0.072 | 0.072 | 0.072 |
| Q/M: | Representative Heat-Removal Rate (W/kg) | 33 | 20 | 19 | 14 |
| Pearly Luster | | A | A | A | A |
| L value | | 46.6 | 40.9 | 46.5 | 37.4 |
| b value | | -4.40 | -3.50 | -5.53 | -3.06 |
| W value (Whiteness) | | 33.4 | 24.9 | 36.3 | 20.5 |
| Dispersion Stability | | B | B | B | B |

Note) The composition ratio is expressed by % by weight.
1) Ester of a mixture of palmitic acid (C16)/stearic acid (C18) = 50/50 (weight ratio) and ethylene glycol, melting point: 61.8°C.
2) The number inside the parenthesis is the number of moles of ethylene oxide.
3) The fatty acid containing 97% by weight of stearic acid and 3% by weight of other fatty acids; melting point: 72.8°C.

[Table 2]

| | | Examples | | | Comparative Examples | |
|---|---|---|---|---|---|---|
| | | 5 | 6 | 7 | 1 | 2 |
| Mixing Apparatus | | AGI HOMO MIXER (2L) | AGI HOMO MIXER (2L) | AGI HOMO MIXER (2L) | AGI HOMO MIXER (2L) | AGI HOMO MIXER (2L) |
| (A) | Fatty Acid Glycol Ester: | | | | | |
| | Methylene Glycol of Difatty Acid (C16/C18 = 50/50[1]) | 18.8 | 17.9 | 18.8 | 18.5 | 18.5 |
| (B) | Fatty Acid Monoalkylolamide: | | | | | |
| | Coconut Oil Fatty Acid Monoethanolamide | 7.5 | 7.5 | 7.5 | 7.5 | 7.5 |
| (C) | Alkyl Sulfate | | | | | |
| | Sodium Polyoxyethylene(2) Lauryl Ether Sulfate[2] | 11.0 | 11.0 | 11.0 | 11.0 | 11.0 |
| (D) | Polyoxyalkylene Nonionic Surfactant: | | | | | |
| | Palyoxyethylene(4) Lauryl Ether[2] [HLB: 9.7] | 4.0 | 4.0 | 4.0 | 4.0 | 4.0 |
| (E) | Other Ingredients | | | | | |
| | Citric Acid Monohydrate | 0.13 | 0.13 | 0.13 | 0.13 | 0.13 |
| | Sodium Benzoate | 1.0 | 1.0 | 1.0 | 1.0 | 1.0 |
| | Water | Balance | Balance | Balance | Balance | Balance |
| (F) | Crystallization Additive | | | | | |
| | Fatty Acid[3] | - | - | - | 1.5 | 1.5 |
| | Aliphatic Alcohol[4] | 1.2 | - | - | - | - |
| | Fatty Acid Monoglyceride (C16/C18 = 25/75[5]) | - | 2.1 | - | - | - |
| | Aliphatic Ether[6] | - | - | 1.2 | - | - |
| M: | Amount of Pearly Luster Composition Formulated (kg) | 2.0 | 2.0 | 2.0 | 2.0 | 2.0 |
| Agitation Rotational Speed (r/min) | | 46 | 46 | 46 | 120 | 46 |
| c: | Specific Heat of Pearly Luster Composition (J/kg/K) | 3,090 | 3,090 | 3,090 | 3,090 | 3,090 |

(continued)

| Mixing Apparatus | | AGI HOMO MIXER (2L) | AGI HOMO MIXER (2L) | AGI HOMO MIXER (2L) | AGI HOMO MIXER (2L) | AGI HOMO MIXER (2L) |
|---|---|---|---|---|---|---|
| U: | Overall Heat-Transfer Coefficient ($W/m^2/K$) | 120 | 120 | 120 | 120 | 120 |
| Setting of Cooling Water | | Cooling at 0.5°C/min | Cooling at 0.5°C/min | Cooling at 0.5°C/min | Cooling at 0.5°C/min and Keeping at 41°C | Cooling at 0.5°C/min |
| Initial Crystallization Temperature (°C) | | 43.7 | 41.1 | 42.0 | 41.2 | 28.6 |
| Terminal Crystallization Temperature (°C) | | 48.7 | 46.8 | 47.2 | 44.4 | 32.6 |
| Tp: | Representative Midpoint Temperature (°C) | 46.2 | 44.0 | 44.6 | 42.8 | 30.6 |
| $Tc_1$: | Coolant Temperature (°C) of Jacket Inlet at Representative Point | 37.4 | 35.2 | 35.9 | 41.1 | 20.6 |
| $Tc_2$: | Coolant Temperature (°C) of Jacket Outlet at Representative Point | 38.3 | 36.1 | 36.9 | 41.0 | 23.3 |
| ΔT: | Average Temperature Difference (K) | 8.3 | 8.3 | 8.2 | 1.8 | 8.6 |
| A: | Heat-Transfer Area ($m^2$) | 0.072 | 0.072 | 0.072 | 0.072 | 0.072 3 |
| Q/M: | Representative Heat-Removal Rate (W/kg) | 36 | 36 | 35 | 8 | 37 |
| Pearly Luster | | A | A | A | A | B |
| L value | | 46.4 | 43.4 | 42.3 | 32.7 | 49.3 |
| b value | | -4.30 | -4.70 | -7.36 | -2.00 | -6.74 |
| W value (Whiteness) | | 32.9 | 30.5 | 35.7 | 14.4 | 43.3 |
| Dispersion Stability | | B | B | B | C | B |

Note) The composition ratio is expressed by % by weight.
1) Ester of a mixture of palmitic acid (C16)/stearic acid (C18) = 50/50 (weight ratio) and ethylene glycol, melting point: G1.8°C.
2) The number inside the parenthesis is the number of moles of ethylene oxide.
3) The fatty acid containing 97% by weight of stearic acid and 3% by weight of other fatty acids; melting point: 72.8°C.
4) The aliphatic alcohol containing 98% by weight of cetyl alcohol and 2% by weight of other higher alochols; melting point: 52.8°C.
5) The fatty acid monoglyceride being a mixture of myristic acid monoglyceride (C16)/stearic acid monoglyceride (C18) = 25/75 (weight ratio); melting point: 72.3°C.
6) The aliphatic ether containing 98% by weight of distearyl ether and 2% by weight of other aliphatic ethers; melting point: 64.0°C.

It can be seen from the above results that the pearly luster compositions obtained in Examples 1 to 7 have

strong pearly luster, high whiteness, and excellent dispersion stability. On the other hand, it can be seen that the pearly luster composition obtained in Comparative Example 1 where a representative heat-removal rate per unit mass during the crystallization is less than 9 [W/kg] has a low whiteness and low dispersion stability even while pearly luster is strong. In addition, it can be seen that the pearly luster composition obtained in Comparative Example 2 where a representative heat-removal rate per unit mass during the crystallization is greater than 36 [W/kg] has a weak pearly luster even while whiteness is high.

Examples 8 to 15

A mixture of a fatty acid monoalkylolamide, an alkyl sulfate, a polyoxyalkylene nonionic surfactant, and other ingredients listed in Tables 3 and 4 was mixed at 80°C with T.K. AGI HOMO MIXER S100 Model (manufactured by PRIMIX Corporation) in which an agitation rotational speed was set at a rate listed in Tables 3 and 4. Thereto were added a fatty acid glycol ester and a crystallization additive which were previously melted and mixed in a molten state, and the mixture obtained was mixed to provide a molten mixture solution. Thereafter, a cooling water set as shown in Tables 3 and 4 was allowed to flow through a jacket to cool the molten mixture solution, to provide a pearly luster composition.

[Table 3]

| | | Examples | | | |
|---|---|---|---|---|---|
| | | 8 | 9 | 10 | 11 |
| Mixing Apparatus | | AGI HOMO MIXER (100L) | AGI HOMO MIXER ( (100L) | AGI HOMO MIXER (100L) | AGI HOMO MIXER (100L) |
| (A) | Fatty Acid Glycol Ester: | | | | |
| | Ethylene Glycol of Difatty Acid (C16/C18 = 50/50[1)]) | 18.8 | 18.8 | 18.5 | 18.5 |
| (B) | Fatty Acid Monoalkylolamide: | | | | |
| | Coconut Oil Fatty Acid Monoethanolamide | 7.5 | 7.5 | 7.5 | 7.5 |
| (C) | Alkyl Sulfate | | | | |
| | Sodium Polyoxyethylene(2) Lauryl Ether Sulfate[2)] | 11.0 | 11.0 | 11.0 | 11.0 |
| (D) | Polyoxyalkylene Nonionic Surfactant: | | | | |
| | Polyoxyethylene(4) Lauryl Ether[2)] [HLB: 9.7] | 4.0 | 4.0 | 4.0 | 4.0 |
| (E) | Other Ingredients: | | | | |
| | Citric Acid Monohydrate | 0.13 | 0.13 | 0.13 | 0.13 |
| | Sodium Benzoate | 1.0 | 1.0 | 1.0 | 1.0 |
| | Water | Balance | Balance | Balance | Balance |

(continued)

| Mixing Apparatus | | AGI HOMO MIXER (100L) | AGI HOMO MIXER ( (100L) | AGI HOMO MIXER (100L) | AGI HOMO MIXER (100L) |
|---|---|---|---|---|---|
| (F) | Crystallization Additive Fatty Acid[3)] | 1.2 | 1.2 | 1.5 | 1.5 |
| M: | Amount of Pearly Luster Composition Formulated (kg) | 100.0 | 100.0 | 100.0 | 100.0 |
| Agitation Rotational Speed (r/min) | | 72 | 72 | 72 | 72 |
| c: | Specific Heat of Pearly Luster Composition (J/kg/K) | 3,090 | 3,090 | 3,090 | 3,090 |
| U: | Overall Heat-Transfer Coefficient ($W/m^2/K$) | 190 | 190 | 190 | 190 |
| Setting of Cooling Water | | Cooling at 0.5°C/min and Keeping at 27°C | Cooling at 0.5°C/min and Keeping at 32°C | Cooling at 0.5°C/min and Keeping at 21°C | Cooling at 0.5°C/min and Keeping at 26°C |
| Initial Crystallization Temperature (°C) | | 34.4 | 36.2 | 31.1 | 32.4 |
| Terminal Crystallization Temperature (°C) | | 43.8 | 45.5 | 41.1 | 41.9 |
| Tp: | Representative Midpoint Temperature (°C) | 39.1 | 40.9 | 36.1 | 37.2 |
| $Tc_1$: | Coolant Temperature (°C) of Jacket Inlet at Representative Point | 27.5 | 32.3 | 21.2 | 26.1 |
| $Tc_2$: | Coolant Temperature (°C) of Jacket Outlet at Representative Point | 29.0 | 33.4 | 21.9 | 27.5 |
| $\Delta T$: | Average Temperature Difference (K) | 10.8 | 8.0 | 14.6 | 10.4 |
| A: | Heat-Transfer Area ($m^2$) | 1.2 | 1.2 | 1.2 | 1.2 |
| Q/M: | Representative Heat-Removal Rate (W/kg) | 25 | 18 | 33 | 24 |
| Pearly Luster | | A | A | A | A |
| L value | | 43.8 | 42.4 | 46.1 | 44.2 |
| b value | | -4.68 | -4.38 | -5.78 | -5.36 |
| W value (Whiteness) | | 30.9 | 28.6 | 36.5 | 33.1 |

(continued)

| | | | | |
|---|---|---|---|---|
| Mixing Apparatus | AGI HOMO MIXER (100L) | AGI HOMO MIXER ( (100L) | AGI HOMO MIXER (100L) | AGI HOMO MIXER (100L) |
| Dispersion Stability | B | B | A | A |

Note) The composition ratio is expressed by % by weight.
1) Ester of a mixture of palmitic acid (C16)/stearic acid (C18) = 50/50 (weight ratio) and ethylene glycol, melting point: 61.8°C.
2) The number inside the parenthesis is the number of moles of ethylene oxide.
3) The fatty acid containing 97% by weight of stearic acid and 3% by weight of other fatty acids; melting point: 72.8°C.

[Table 4]

| | | Examples | | | |
|---|---|---|---|---|---|
| | | 12 | 13 | 14 | 15 |
| Mixing Apparatus | | AGI HOMO MIXER (100L) | AGI HOMO MIXER (100L) | AGI HOMO MIXER (100L) | AGI HOMO MIXER (100L) |
| (A) | Fatty Acid Glycol Ester: | | | | |
| | Methylene Glycol of Difatty Acid (C16/C18 = 50/50[1]) | 18.5 | 18.2 | 18.2 | 17.9 |
| (B) | Fatty Acid Monoalkylolamide: | | | | |
| | Coconut Oil Fatty Acid Monoethanolamide | 7.5 | 7.5 | 7.5 | 7.5 |
| (C) | Alkyl Sulfate | | | | |
| | Sodium Polyoxyethylene(2) Lauryl Ether Sulfate[2] | 11.0 | 11.0 | 11.0 | 11.0 |
| (D) | Polyoxyalkylene Nonionic Surfactant: | | | | |
| | Polyoxyethylene(4) Lauryl Ether[2] [HLB: 9.7] | 4.0 | 4.0 | 4.0 | 4.0 |
| (E) | Other Ingredients: | | | | |
| | Citric Acid Monohydrate | 0.13 | 0.13 | 0.13 | 0.13 |
| | Sodium Benzoate | 1.0 | 1.0 | 1.0 | 1.0 |
| | Water | Balance | Balance | Balance | Balance |
| (F) | Crystallization Additive | | | | |
| | Fatty Acid[3] | 1.5 | 1.8 | 1.8 | 2.1 |

(continued)

| Mixing Apparatus | | AGI HOMO MIXER (100L) | AGI HOMO MIXER (100L) | AGI HOMO MIXER (100L) | AGI HOMO MIXER (100L) |
|---|---|---|---|---|---|
| M: | Amount of Pearly Luster Composition Formulated (kg) | 100.0 | 100.0 | 100.0 | 100.0 |
| Agitation Rotational Speed (r/min) | | 72 | 72 | 72 | 36 |
| c: | Specific Heat of Pearly Luster Composition (J/kg/K) | 3,090 | 3,090 | 3,090 | 3,090 |
| U: | Overall Heat-Transfer Coefficient ($W/m^2/K$) | 190 | 190 | 190 | 190 |
| Setting of Cooling Water | | Cooling at 0.5°C/min and Keeping at 34°C | Cooling at 0.5°C/min and Keeping at 27°C | Cooling at 0.5°C/min and Keeping at 32°C | Cooling at 0.5°C/min and Keeping at 32°C |
| Initial Crystallization Temperature (°C) | | 37.0 | 31.5 | 35.1 | 34.2 |
| Terminal Crystallization Temperature (°C) | | 46.8 | 41.0 | 45.0 | 39.7 |
| Tp: | Representative Midpoint Temperature (°C) | 41.9 | 36.3 | 40.1 | 37.0 |
| $Tc_1$: | Coolant Temperature (°C) of Jacket Inlet at Representative Point | 34.3 | 27.0 | 32.3 | 32.3 |
| $Tc_2$: | Coolant Temperature (°C) of Jacket Outlet at Representative Point | 36.3 | 28.8 | 33.8 | 33.7 |
| ΔT: | Average Temperature Difference (K) | 6.5 | 8.4 | 7.0 | 4.0 |
| A: | Heat-Transfer Area ($m^2$) | 1.2 | 1.2 | 1.2 | 1.2 |
| Q/M: | Representative Heat-Removal Rate (W/kg) | 15 | 19 | 16 | 9 |
| Pearly Luster | | A | A | A | A |
| L value | | 41.7 | 45.2 | 42.1 | 40.3 |
| b value | | -3.00 | -5.54 | -4.68 | -5.89 |
| W value (Whiteness) | | 24.5 | 34.7 | 29.0 | 29.8 |

(continued)

| | | | | |
|---|---|---|---|---|
| Mixing Apparatus | AGI HOMO MIXER (100L) | AGI HOMO MIXER (100L) | AGI HOMO MIXER (100L) | AGI HOMO MIXER (100L) |
| Dispersion Stability | B | A | B | B |

Note) The composition ratio expressed by % by weight.
1) Ester of a mixture of palmitic acid (C16)/stearic acid (C18) = 50/50 (weight ratio) and ethylene glycol melting point: 61.8°C.
2) The number inside the parenthesis is the number of moles of ethylene oxide.
3) The fatty acid containing 97% by weight of stearic acid and 3% by weight of other fatty acids; melting point: 72.8°C.

It can be seen from the above results that even when the production scale is increased, a pearly luster composition having a strong pearly luster, a high whiteness, and excellent dispersion stability is obtained according to the methods of Examples 8 to 15.

INDUSTRIAL APPLICABILITY

The pearly luster composition obtained by the method of the present invention can be suitably used for shampoos, conditioners, body shampoos, liquid detergents, and the like.

**Claims**

**1.** A method for producing a pearly luster composition comprising a fatty acid glycol ester, a surfactant, and water, and further comprising as a crystallization additive any one selected from the group consisting of (1) a fatty acid, (2) an aliphatic alcohol, (3) a fatty acid monoglyceride, and (4) an aliphatic ether, the method comprising the step of cooling a molten mixture solution comprising the fatty acid glycol ester, the surfactant, water, and the crystallization additive, wherein a representative heat-removal rate per unit mass during crystallization in the cooling step is from 9 to 36 [W/kg].

**2.** The method for producing a pearly luster composition according to claim 1, wherein the crystallization additive comprises the fatty acid, and the surfactant comprises a polyoxyalkylene nonionic surfactant.

**3.** The method for producing a pearly luster composition according to claim 1, wherein the crystallization additive comprises the aliphatic alcohol, and the surfactant comprises a polyoxyalkylene nonionic surfactant.

**4.** The method for producing a pearly luster composition according to claim 1, wherein the crystallization additive comprises the fatty acid monoglyceride or the aliphatic ether.

**INTERNATIONAL SEARCH REPORT**

International application No.
PCT/JP2009/065939

A. CLASSIFICATION OF SUBJECT MATTER
*C11D11/00*(2006.01)i, *A61K8/33*(2006.01)i, *A61K8/37*(2006.01)i, *A61K8/39*(2006.01)i, *A61Q5/02*(2006.01)i, *A61Q19/10*(2006.01)i, *C11D1/72*(2006.01)i, *C11D3/20*(2006.01)i
According to International Patent Classification (IPC) or to both national classification and IPC

B. FIELDS SEARCHED
Minimum documentation searched (classification system followed by classification symbols)
C11D1/00-17/08, A61K8/00-8/99

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 9-111291 A (Kao Corp.), 28 April 1997 (28.04.1997), entire text (Family: none) | 1-4 |
| Y | JP 2007-162002 A (Kao Corp.), 28 June 2007 (28.06.2007), entire text (Family: none) | 1-4 |
| Y | JP 2003-506393 A (Cognis Deutschland GmbH & Co., KG.), 18 February 2003 (18.02.2003), entire text & WO 2001/010403 A1 & EP 1200062 A1 & DE 19937298 A1 | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
27 November, 2009 (27.11.09)

Date of mailing of the international search report
08 December, 2009 (08.12.09)

Name and mailing address of the ISA/
Japanese Patent Office

Facsimile No.

Authorized officer

Telephone No.

Form PCT/ISA/210 (second sheet) (April 2007)

**INTERNATIONAL SEARCH REPORT**

International application No.
PCT/JP2009/065939

C (Continuation). DOCUMENTS CONSIDERED TO BE RELEVANT

| Category* | Citation of document, with indication, where appropriate, of the relevant passages | Relevant to claim No. |
|---|---|---|
| Y | JP 11-503723 A (Henkel KGaA), 30 March 1999 (30.03.1999), entire text & WO 1996/029981 A1 & US 6306916 B1 & EP 817609 A1 & DE 19511571 A1 | 1-4 |
| P,X | WO 2008/126558 A1 (Kao Corp.), 23 October 2008 (23.10.2008), entire text & JP 2008-255110 A & JP 2009-19194 A | 1-4 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI6504781 A
- JP 2000212031 A
- JP 2000511913 A
- JP 2003506393 A

### Non-patent literature cited in the description

- Kagaku Kogaku Gairon. Sangyo Tosho K.K, 1993, 66